# EUROPÄISCHE PATENTANMELDUNG

(11) **EP 1 566 172 A1**
(43) Veröffentlichungstag der Anmeldung: **24.08.2005**
(21) Anmeldenummer: 05100779.7
(22) Anmeldetag: 04.02.2005
(51) Int. Cl.: A61K 7/50

(54) **Stabile Kosmetika enthaltend vorgelatinisierte quervernetzte Stärkederivate**

(30) Priorität: 23.02.2004 DE 102004009149
(71) Anmelder: Beiersdorf AG, 20253 Hamburg (DE)
(72) Erfinder: Dörschner, Albrecht, 20146, Hamburg (DE); Ruppert, Stephan, 20259, Hamburg (DE); Jensen, Ursula, 20253, Hamburg (DE); Willems, Elke, 20251, Hamburg (DE); Frese, Christian, 22765, Hamburg (DE); Kauffeldt, Martin, 22307, Hamburg (DE)

(57) **Zusammenfassung**

Kosmetische Zubereitung enthaltend einen oder mehrere labile Inhaltsstoffe sowie ein oder mehrere vorgelatinisierte, quervernetzte Stärkederivate.

## Beschreibung

Die vorliegende Erfindung betrifft eine kosmetische Zubereitung enthaltend einen oder mehrere labile Inhaltsstoffe und ein oder mehrere vorgelatinisierte, quervernetzte Stärkederivate.

Der Wunsch nach sauberer Haut ist wohl so alt wie die Menschheit, denn Schmutz, Schweiß und Reste abgestorbener Hautpartikel bieten den idealen Nährboden für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neuentwickelten synthetischen Tensiden formuliert werden konnten. Baden und Duschen sind seitdem aus unserem täglichen Leben nicht mehr wegzudenken. Den Verbrauchern stehen heutzutage eine Vielzahl von Produkten für die Reinigung der verschiedenen Körperpartien zur Verfügung.

Eine besondere Gruppe an Hautreinigungsprodukten bilden dabei die Gesichtsreinigungsprodukte. Da die Gesichtshaut besonders empfindlich ist, werden für die Gesichtsreinigung besonders milde und die Haut nicht reizende Produkte eingesetzt. Meist werden dabei Gele, das heißt halbfeste, mehr oder weniger transparente Systeme verwendet.

Reinigungsgele enthalten als Hauptbestandteile Wasser, Tenside und Verdicker (Gelbildner).

Die Tenside stellen in den Reinigungsgelen die waschaktive Substanzen dar. Sie sorgen, bedingt durch ihren spezifischen Molekülaufbau mit jeweils einer hydrophilen (wasseranziehenden) und hydrophoben (wasserabweisenden) Gruppierung im gleichen Molekül, für die Herabsetzung der Oberflächenspannung des Wassers, wodurch die Schmutzentfernung erleichtert wird. Man unterscheidet je nach Ladungszustand zwischen anionischen, kationischen, amphoteren und nichtionischen Tensiden. Aufgrund ihrer Fähigkeit, die Oberflächespannung von Wasser zu reduzieren, bewirken Tenside ein Aufschäumen der Zubereitung.

Die Verdicker, auch Gelbildner genannt, bilden in dem Reinigungsgel ein dreidimensionales Netzwerk aus, in dem die Flüssigkeit (in der Regel Wasser) immobilisiert ist. Als Verdicker werden meist Polymere wie Polyacrylate eingesetzt. Diese werden der zu verdickenden Zubereitung bei neutralem pH-Wert zugesetzt und anschließend durch die Zugabe von Basen deprotoniert, wodurch die Zubereitung in ein viskoses Gel übergeht.

Darüber hinaus können Reinigungsgele eine Reihe weiterer Inhaltsstoffen enthalten, beispielsweise Parfümstoffe oder kosmetische Wirk- und Pflegestoffe.

Herkömmliche, nach dem Stand der Technik hergestellte Reinigungsgele haben jedoch den Nachteil, dass sie keine labilen Inhaltsstoffe enthalten können, insbesondere wenn sie in transparenten (d.h. klar durchsichtigen) oder transluzenten (d.h. milchig durchscheinenden) Verpackungen aufbewahrt werden. Insbesondere transparente Verpackungen stellen jedoch eine für Gele besonders bevorzugte Verpackungsform dar, da durch diese Art der Verpackung die in den Gelen vorteilhaft enthaltenen Farb- und/oder Effektstoffe optisch voll zur Geltung kommen können. Nach dem Stand der Technik konnte dieses Problem allenfalls dadurch gelöst werden, dass man der Zubereitung oder dem Verpackungsmaterial UV-Lichtschutzfilter beifügte (wodurch u.a. die Herstellungskosten der Kosmetika ansteigen) oder auf (photo-) labile Inhaltsstoffe verzichtete.

Es war daher die Aufgabe der vorliegenden Erfindung, die Mängel des Standes der Technik zu beseitigen.

Überraschend gelöst wurde die Aufgabe nun durch eine kosmetische Zubereitung enthaltend ein oder mehrere labile Inhaltsstoffe sowie ein oder mehrere vorgelatinisierte, quervernetzte Stärkederivate.

Ferner wurde die Aufgabe überraschend gelöst durch ein Kosmetikum, welches gebildet wird aus
a) einer transparenten oder transluzenten Verpackung und
b) einer kosmetischen Zubereitung enthaltend einen oder mehrere labile Inhaltsstoffe sowie ein oder mehrere vorgelatinisierte, quervernetzte Stärkederivate.

Außerdem wurde die Aufgabe für den Fachmann in nicht nahe liegender Weise gelöst durch die Verwendung von vorgelatinisierten, quervernetzten Stärkederivaten zur Stabilisierung von labilen Inhaltsstoffen in Kosmetika.

Die Erfindung war insbesondere deshalb überraschend, da vorgelatinisierte, quervernetzte Stärkederivate an sich keine nennenswerten UV-Lichtschutzfilter-Eigenschaften aufweisen.

Zwar beschreibt der Stand der Technik in der WO 98/01109 die die Erhöhung der Geruchs- und Lagerstabilität durch Stärkederivate, doch konnte diese Schrift nicht den Weg zur vorliegenden Erfindung, da es sich bei den in der WO 98/01109 beschriebenen Effekten um eine Erhöhung der Thermostabilität bzw. um eine Erhöhung der mikrobiellen Stabilität handelt. Wie jedoch dem Chemiker bekannt ist, liegen thermischen und photochemischen Reaktionen vollkommen unterschiedliche Reaktionsmechanismen zu Grunde, so dass von einer thermischen Stabilität eines Stoffes nicht auf dessen Photostabilität geschlossen werden kann (und umgekehrt).

Auch die Offenbarungen der DE 102 16 504, WO 96/22073 und US 6248338 konnten nicht den Weg zur vorliegenden Erfindung weisen.

Es wird erfindungsgemäß unter "Stabilität" insbesondere die farbliche Stabilität der Zubereitungen verstanden. Die erfindungsgemäßen Zubereitungen und Verwendungen führen insbesondere dazu, dass sich die Zubereitungen unter Lichteinfluss nicht mehr verfärben.

Es werden erfindungsgemäß unter labilen Inhaltsstoffen Verbindungen verstanden, die sich im Laufe der Zeit verfärben und somit zu einer Verfärbung (Farbänderung) der sie enthaltenden Zubereitung führen. Insbesondere werden unter labilen Inhaltsstoffen Verbindungen verstanden, die sich unter Lichteinfluss verfärben (d.h. photolabil sind) und damit zu einer Verfärbung (Farbänderung) der sie enthaltenden Zubereitung führen.

Es ist erfindungsgemäß vorteilhaft, wenn als vorgelatinisierte, quervernetzte Stärkederivate hydroxypropylierte Phosphatester eingesetzt werden. Insbesondere vorteilhaft sind solche Stärkederivate, wie sie in der US 6248338 beschrieben werden, besonders vorteilhaft Hydroxypropyldistärkephosphat. Ganz besonders bevorzugt ist dabei der Einsatz eines Hydroxypropyldistärkephosphates, wie es als Produkt Structure® XL der Firma National Starch verkauft wird.

Erfindungsgemäß vorteilhafte Zubereitungen, Kosmetika oder Verwendungen sind dadurch gekennzeichnet, dass die vorgelatinisierten, quervernetzten Stärkederivate in einer Gesamtkonzentration von 0,05 bis 5 Gewichts-% und bevorzugt in einer Gesamtkonzentration von 0,1 bis 2 Gewichts-%, jeweils bezogen auf das Gesamtgewicht der Zubereitung in dieser enthalten sind.

In einer erfindungsgemäß bevorzugten Ausführungsform sind erfindungsgemäß vorteilhafte Zubereitungen, Kosmetika oder Verwendungen dadur ch gekennzeichnet, dass als labile Inhaltsstoffe Licochalkon A und/oder Ubichinon Q-10 eingesetzt werden.

Dabei ist es erfindungsgemäß vorteilhaft, wenn die Gesamtmenge an Licochalkon A und Ubichinon Q-10 in der Zubereitung von 0,001 bis 0,3 Gewichts-%, und erfindungsgemäß bevorzugt, wenn die Gesamtmenge an Licochalkon A und Ubichinon Q-10 in der Zubereitung von 0,002 bis 0,2 Gewichts-% bezogen auf das Gesamtgewicht der Zubereitung beträgt.

Erfindungsgemäß vorteilhaft beträgt die Konzentration an Licochalkon A in der Zubereitung von 0,001 bis 0,1 Gewichts-% und erfindungsgemäß bevorzugt von 0,002 bis 0,05 Gewichts-%, jeweils bezogen auf das Gesamtgewicht der Zubereitung.

Licochalkon A ist ein wirksamer Bestandteil des wässrigen Auszuges von *Glycyrrhiza inflata*.

Die Pflanzenart *Glycyrrhiza inflata* gehört wie das in Europa offizinelle Süßholz *Glycyrrhiza glabra* der Gattung *Glycyrrhiza* an, die zur Pflanzenfamilie der Fabaceae (Erbsengewächse) gehört. Die Droge *Radix Glycyrrhizae inflatae*, d.h., die Wurzel der Pflanze, ist, beispielsweise in der fernöstlichen Medizin, gebräuchlich.

Ein Bestandteil des wäßrigen Auszugs aus *Radix Glycyrrhizae inflatae* ist das Licochalkon A, Vorteilhaft im Sinne der vorliegenden Erfindung ist es daher, das erfindungsgemäße Licochalkon A in Form des wässrigen Auszuges von *Radix Glycyrrhizae inflatae* der erfindungsgemäßen Zubereitung zuzusetzen.

Erfindungsgemäß vorteilhaft beträgt die Konzentration an Ubichinon Q-10 in der Zubereitung von 0,001 bis 0,1 Gewichts-% und erfindungsgemäß bevorzugt von 0,002 bis 0,05 Gewichts-%, jeweils bezogen auf das Gesamtgewicht der Zubereitung.

In einer weiteren erfindungsgemäß bevorzugten Ausführungsform sind erfindungsgemäß vorteilhafte Zubereitungen, Kosmetika oder Verwendungen dadurch gekennzeichnet, dass es sich bei den labilen Inhaltsstoffen um Parfümstoffe handelt.

Dabei ist es erfindungsgemäß vorteilhaft, wenn die Gesamtmenge an Parfümstoffen in der Zubereitung von 0 bis 2,0 Gewichts-%, und erfindungsgemäß bevorzugt, wenn die Gesamtmenge an Parfümstoffen in der Zubereitung von 0 bis 0,8 Gewichts-%, jeweils bezogen auf das Gesamtgewicht der Zubereitung beträgt.

Auch Mischungen aus Licochalkon A, Ubichinon Q-10 und Parfümstoffen können erfindungsgemäß vorteilhaft in allen Mischungsverhältnissen (zweier oder dreier Komponenten) in der erfindungsgemäßen Zubereitung oder Verwendung eingesetzt werden.

Die erfindungsgemäßen Zubereitungen können darüber hinaus eine Vielzahl weiterer kosmetischer Wirk-, Hilfs- und Zusatzstoffe enthalten.

Erfindungsgemäß vorteilhaft handelt es sich bei der erfindungsgemäßen Zubereitung bzw. bei der Zubereitung, in der die erfindungsgemäße Verwendung verwirklicht ist, um eine Zubereitung auf Wasserbasis. Erfindungsgemäß vorteilhaft ist dabei eine Wasserkonzentration von 60 bis 90 Gewichts-%, bevorzugt von 70 bis 90 Gewichts-% und ganz besonders bevorzugt von 75 bis 85 Gewichts-%, jeweils bezogen auf das Gesamtgewicht der Zubereitung.

Erfindungsgemäß vorteilhaft handelt es sich bei der erfindungsgemäßen Zubereitung bzw. bei der Zubereitung, in der die erfindungsgemäße Verwendung verwirklicht ist, um eine tensidhaltige Zubereitung.

Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Die folgenden Tenside können erfindungsgemäß vorteilhaft eingesetzt werden:

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Erfindungsgemäß vorteilhaft beträgt die Gesamtkonzentration an Tensiden in den erfindungsgemäßen Zubereitungen von 0,5 bis 15 Gewichts-% und erfindungsgemäß bevorzugt von 2 bis 10 Gewichts-%, jeweils bezogen auf das Gesamtgewicht der Zubereitung.

Die erfindungsgemäß besonders bevorzugten Tensidmischungen und ihre besonders bevorzugten Einsatzkonzentrationen sind in den Beispielrezepturen offenbart.

Erfindungsgemäße Zubereitungen können auch vorteilhaft Verdicker enthalten. Geeignete Verdicker sind:
Homopolymere der Acrylsäure mit einem Molekulargewicht von 2000000 bis 6000000 wie z.B. Handelsprodukt Carbopole. Weiter Verdicker werden vertrieben unter den Namen Carbopol 940, Carbopol EDTA 2001 oder Modarez V 600 PX.
Polymere aus Acrylsäure und Acrylamid (Natriumsalz) mit einem Molekulargewicht von 2000000 bis 6000000 wie z.B. Hostacerin PN 73 oder das unter dem Namen Amigel vertriebene Sclerotium Gum.
Außerdem geeignet sind Copolymere der Acrylsäure oder der Methacrylsäure wie z.B. Carbopol 1342 oder Permulen TRI.
Weitere Verdickertypen sind Polyglycole, Cellulosederivate, insbesondere Hydroxyalkylcellulosen sowie Alginate, Carageenan und anorganische Verdicker wie z.B. natürliche oder synthetische Bentonite.

Erfindungsgemäß vorteilhaft beträgt die Gesamtkonzentration an Verdickern in den erfindungsgemäßen Zubereitungen von 0,01 bis 2 Gewichts-% und erfindungsgemäß bevorzugt von 0,1 bis 1 Gewichts-%, jeweils bezogen auf das Gesamtgewicht der Zubereitung.

Die erfindungsgemäß besonders bevorzugten Verdicker und ihre besonders bevorzugten Einsatzkonzentrationen sind in den Beispielrezepturen offenbart.

Die erfindungsgemäße Zubereitung enthält vorteilhafter Weise einen oder mehrere Konditionierer. Erfindungsgemäß bevorzugte Konditionierer sind beispielsweise alle Verbindungen, welche im *International Cosmetic Ingredient Dictionary and Handbook* (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar. So sind beispielsweise Polyquaternium-1 bis Polyquaternium-56 aber auch die Polyethylenglycole und Polyproylenglycole erfindungsgemäß vorteilhafte Konditionierungsmittel.

Die erfindungsgemäße Zubereitung kann in vorteilhafter Weise Abrasiva mit einer durchschnittlichen Partikelgröße kleiner 400 µm, in einer Menge von 0,1 bis 3 Gewichts-%, jeweils bezogen auf das Gesamtgewicht der Zubereitung enthalten, wobei die Abrasiva vorzugsweise aus Polyethylen bestehen.

Die erfindungsgemäßen kosmetischen Zubereitungen können eine Reihe von Pigmenten enthalten.
Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe *Colour Index,* 3. *Auflage, Society of Dyers and Colourists*, *Bradford*, *England*, 1971 entnommen.

| **Chemische oder sonstige Bezeichnung** | **CIN** | **Farbe** |
|---|---|---|
| Pigment Green | 10006 | Grün |
| Acid Green 1 | 10020 | Grün |
| 2,4-Dinitrohydroxynaphthalin-7-sulfosäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'-sulfosäurediethylamid-1'-phenylazo)-3-hy- | 12490 | Rot |
| droxy-5"-chloro-2",4"-dimethoxy-2-naphthoesäureanilid | | |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin- | 14700 | Rot |
| 4-sulfosäure | | |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2- | 15525 | Rot |
| hydroxynaphthalin | | |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | Rot |
| 1-(2-Sulfo-4-methyl-5-chlor-1-phenylazo)-2-hydrooy- | 15865 | Rot |
| naphthalin-3-carbonsäure | | |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2 -phenylazo- 1 -naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy- | 19140 | Gelb |
| pyrazolon-3-carbonsäure | | |
| Pigment Yellow 16 | 20040 | Gelb |
| 2,6-(4'-Sulfo-2", 4"-dimethyl)-bis-phenylazo)1,3-dihydroxy- | 20170 | Orange |
| benzol | | |
| Acid Black 1 | 20470 | Schwar |
| | | z |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1- | 27755 | Schwarz |
| hydroxy-7-aminonaphthalin-3,6-disulfosäure | | |
| 4'-[(4"-Sulfo-1"-phenylazo)-7'-sulfo-1'-naphthylazo]-1-hydroxy- | 28440 | Schwarz |
| 8-acetyl-aminonaphthalin-3,5-disulfosäure | | |
| Direct Orange 34, 39, 44, 46, 60 | 40215 | Orange |
| Food Yellow | 40800 | Orange |
| trans-β-Apo-8'-Carotinaldehyd (C₃₀) | 40820 | Orange |
| trans-Apo-8'-Carotinsäure (C₃₀)-ethylester | 40825 | Orange |
| Canthaxanthin | 40850 | Orange |
| Acid Blue 1 | 42045 | Blau |
| 2,4-Disulfo-5-hydroxy-4'-4"-bis-(diethylamino)triphenyl- | 42051 | Blau |
| carbinol | | |
| 4-[(-4-N-Ethyl-p-sulfobenzylamino)-phenyl-(4-hydroxy-2-sulfo- | 42053 | Grün |
| phenyl)-(methylen)-1-(N-ethylN-p-sulfobenzyl)-2,5- | | |
| cyclohexadienimin] | | |
| Acid Blue 7 | 42080 | Blau |
| (N-Ethyl-p-sulfobenzyl-amino)-phenyl-(2-sulfophenyl)- | 42090 | Blau |
| methylen-(N-ethyl-N-p-sulfo-benzyl)Δ^{2,5}-cyclohexadienimin | | |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violett |
| Basic Violet 2 | 42520 | Violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | Blau |
| 4'-(N-Dimethyl)-amino-4"-(N-phenyl)-aminonaphtho-N-dimethyl-fuchsonimmonium | 44045 | Blau |
| 2-Hydroxy-3,6-disulfo-4,4'-bis-dimethylamino-naphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violett |
| Acid Red 50 | 45220 | Rot |
| Phenyl-2-oxyfluoron-2-carbonsäure | 45350 | Gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydrooynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 8-Amino-2 - phenylazo- 1-naphthol-3,6-disulfosäure, Aluminiumsalz der 4-(4-Sulfo-1-phenylazo)-1-(4-sulfophenyl)-5-hydroxy-pyrazolon-3-carbonsäure, 4'-[(4"-Sulfo-1 "-phenylazo)-7'-sulfo-1'-naphthylazo]-1-hydroxy-8-acetyl-aminonaphthalin-3,5-disulfosäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, 4,4'-Dimethyl-6,6'-dichlorthioindigo, Komplexsalz (Na, Al, Ca) der Karminsäure, rotes und schwarzes Eisenoxid (CIN: 77 491 (rot) und 77 499 (schwarz)), Eisenoxidhydrat (CIN: 77 492), Manganammoniumdiphosphat (CIN 77745), Ultramarin (CIN 77007) und Titandioxid.

Die Zusammensetzungen enthalten gemäß der Erfindung außer den vorgenannten Substanzen gegebenenfalls die in der Kosmetik üblichen Zusatzstoffe, beispielsweise Farbstoffe, antimikrobielle Stoffe, rückfettende Agentien, Komplexierungs- und Sequestrierungsagentien, Perlglanzagentien, Pflanzenextrakte, Vitamine, Wirkstoffe, Antischuppenwirkstoffe, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, UV-Lichtschutzfilter, Schaumstabilisatoren, Elektrolyte, organische Lösemittel oder Silikonderivate.

Kosmetische Reinigungsgele gemäß der Erfindung können kosmetische Hilfsstoffe enthalten, wie sie üblicherweise in solchen Zubereitungen verwendet werden, z. B. Konservierungshelfer, Komplexbildner, Antioxidantien, Puffer, Lösungsvermittler, Dispergiermittel, Bakterizide, Parfüme, weitere Substanzen zum Verhindern oder Steigern des Schäumens, Farbstoffe, Pigmente, die eine färbende Wirkung haben, Verdickungsmittel, Perlglanzpigmente, weichmachende, anfeuchtende und/oder feuchhaltende Substanzen (z.B. Harnstoff), Fette, Öle, Wachse oder andere übliche Bestandteile einer kosmetischen oder dermatologischen Formulierung wie Alkohole, Polyole, Polymere, Omega-Fettsäuren, Schaumstabilisatoren, Elektrolyte, organische Lösungsmittel oder Silikonderivate.

Insbesondere ist es erfindungsgemäß von Vorteil der erfindungsgemäßen Zubereitung Vitamine und Pflanzenextrakte zuzusetzen. So ist beispielsweise der Zusatz von Calcium-Vitamin-Komplexen, Niacinamid und/oder Panthenol erfindungsgemäß besonders vorteilhaft.

Ein weiterer erfindungsgemäß vorteilhafter Wirkstoff stellt beispielsweise Polidocanol dar.

Ferner ist es im Sinne der vorliegenden Erfindung, der erfindungsgemäßen Zubereitung Perlglanzpigmente, Glimmer, Glitterstoffe, Kügelchen und/oder Effektpigmente, Peelingpartikel, wie z.B. Polyethylen, zuzusetzen, um die Zubereitung optisch attraktiver zu gestalten und/oder einen Zusatznutzen herbeizuführen. Auch kann eine erfindungsgemäße Zubereitung in Form eines Gels mit Gasblasen, insbesondere Luftblasen, oder Schlieren, insbesondere Farbschlieren, in für die Erfindung vorteilhafter Weise versehen sein.

Die Zubereitung kann zusätzlich Abrasiva enthalten, z.B. Polymerkügelchen oder -pulver aus Polyethylen, Polypropylen oder anorganischen Oxiden oder Silikaten. Diese haben erfindungsgemäß eine durchschnittlichen Partikelgröße kleiner 400 µm, bevorzugt kleiner 300 µm, besonders bevorzugt zwischen 250 bis 75 µm, in einer Menge von 0,1 bis 3 Gewichts-%, bezogen auf das Gesamtgewicht der Formulierung einzeln oder als Mischung mehrer Abrasiva.

Erfindungsgemäß vorteilhaft weist die erfindungsgemäße Zubereitung bzw. bei der Zubereitung, in der die erfindungsgemäße Verwendung verwirklicht ist, einen pH-Wert von pH 4 bis pH 7 auf.

Erfindungsgemäß vorteilhaft handelt es sich bei der erfindungsgemäßen Zubereitung bzw. bei der Zubereitung, in der die erfindungsgemäße Verwendung verwirklicht ist, um ein Reinigungsgel. Dabei sind transparente Reinigungsgele erfindungsgemäß bevorzugt.

Erfindungsgemäß vorteilhaft handelt es sich bei der erfindungsgemäßen Zubereitung bzw. bei der Zubereitung, in der die erfindungsgemäße Verwendung verwirklicht ist um ein Reinigungsgel zur Reinigung der Haut von Gesicht und Körper. Erfindungsgemäß besonders bevorzugt sind dabei Gele zur Reinigung der Gesichtshaut (sowohl der trockenen Haut, der normalen Mischhaut als auch der fettig-öligen und zur Akne neigenden Haut). Aber auch in Haarshampoos kann die vorliegende Erfindung erfindungsgemäß vorteilhaft verwirklicht werden.

Es ist vorteilhaft im Sinne der vorliegenden Erfindung, wenn das erfindungsgemäße kosmetische Reinigungsgel eine Viskosität von 4000 bis 8000 m Pa s (Viskotester VT 02 der Gesellschaft Haake, Temperatur: 25 °C, Spindel: Drehkörper 1 (24 mm Durchmesser), Rotorgeschwindigkeit: 62,5 min⁻¹) ermittelt.

Die erfindungsgemäße Verpackung kann ein Verpackungsbehältnis aus Glas oder Kunststoff sein. Dabei sind Kunststoffbehältnisse erfindungsgemäß bevorzugt. (PE, PP, PET). Erfindungsgemäß vorteilhaft ist es ferner, wenn das Verpackungesbehältnis nicht eingefärbt sondern farblos ist. Erfindungsgemäß bevorzugt sind transparente Verpackungen.

Die folgenden Beispiele sollen die erfindungsgemäßen Zusammensetzungen erläutern, ohne dass aber beabsichtigt ist, die Erfindung auf diese Beispiele zu beschränken. Die Zahlenwerte in den Beispielen bedeuten Gewichtsprozente, bezogen auf das Gesamtgewicht der jeweiligen Zubereitungen.

### Beispiele

### Duschgele:

| | **1** | **2** | **3** | **4** | **5** |
|---|---|---|---|---|---|
| Natrium Laurethsulfat | 13,2% | 11% | 9,5% | 11% | 9,5% |
| Cocoamidopropylbetain | 1,65% | 3,3% | 3,8% | 3,3% | 3,8% |
| Natriumcocoylglutamat | 1,25% | 0,75% | 2,5% | 0,75% | 0,75% |
| Hydroxypropyldistärkephosphat (Structure® XL) | 0,2% | 0,5% | 1% | 0,2% | 1% |
| PEG-7 Glyceryl Cocoate | --- | --- | --- | 2,0 % | 2,0 % |
| PEG-40 hydriertes Rizinusöl | 0,50% | 0,50% | 0,5% | 0,50% | 0,50% |
| Ubiquinon | --- | 0.01 % | --- | ---- | 0,025% |
| Licochalcone A | 0.025% | --- | 0.05% | 0.02% | 0.02% |
| Polyquaternium-10 | 0,2% | --- | 0,2% | --- | --- |
| Natriumbenzoat | 0,45% | 0,45% | 0,45% | 0,45% | 0,45% |
| Natriumsalicylat | 0,20% | 0,20% | 0,2% | 0,20% | 0,20% |
| Citronensäure | 0,50% | 0,50% | 0,5% | 0,50% | 0,50% |
| Parfum | q.s. | q.s. | q.s. | q.s. | q.s. |
| Wasser | ad 100 | ad 100 | ad 100 | ad 100 | ad 100 |

### LES-freie Duschgele

| | **1** | **2** | **3** |
|---|---|---|---|
| Natrium Myrethsulfat | 5% | 4% | 6% |
| Laurylglucosid | 2,5% | --- | --- |
| Decylglucosid | --- | 3% | --- |
| Natrium Cocoamphoacetat | 6,5% | 7% | 8% |
| Hydroxypropyldistärkephosphat (Structure® XL) | 0,5% | 0,2% | 1% |
| PEG-200 hydriertes Glycerylpalmitat | 0,4% | 0,4% | 0,4% |
| PEG-40 hydriertes Rizinusöl | 1 % | 1 % | 1 % |
| Diammonium Citrat | 0,12% | 0,12% | 0,12% |
| Polyquaternium-10 | 0,2% | --- | --- |
| Licochalcone A | 0.025% | -- | 0.05% |
| Ubiquinone | 0,01 % | 0,025% | -- |
| Natriumbenzoat | 0,3% | 0,3% | 0,3% |
| Natriumsalicylat | 0,2% | 0,2% | 0,2% |
| Citronensäure | 1,2% | 1,2% | 1,2% |
| Parfum | q.s. | q.s. | q.s. |
| Wasser | ad 100 | ad 100 | ad 100 |

**Gesichtsreinigungsgele**

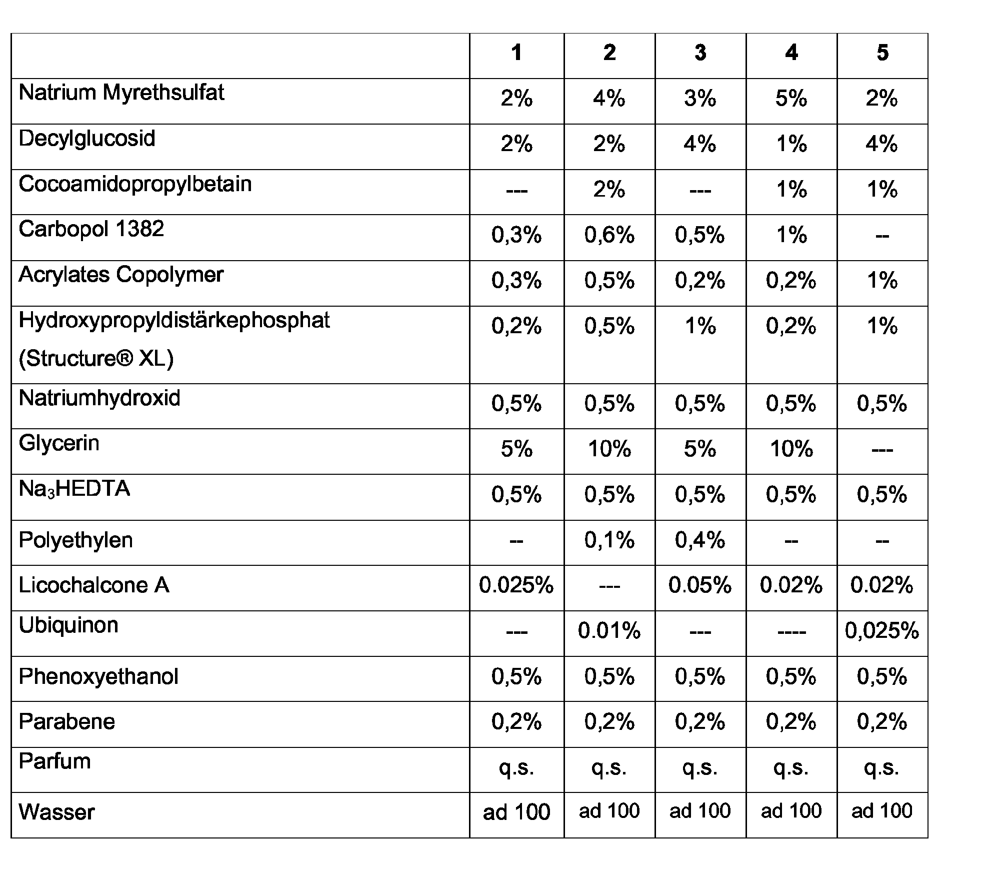

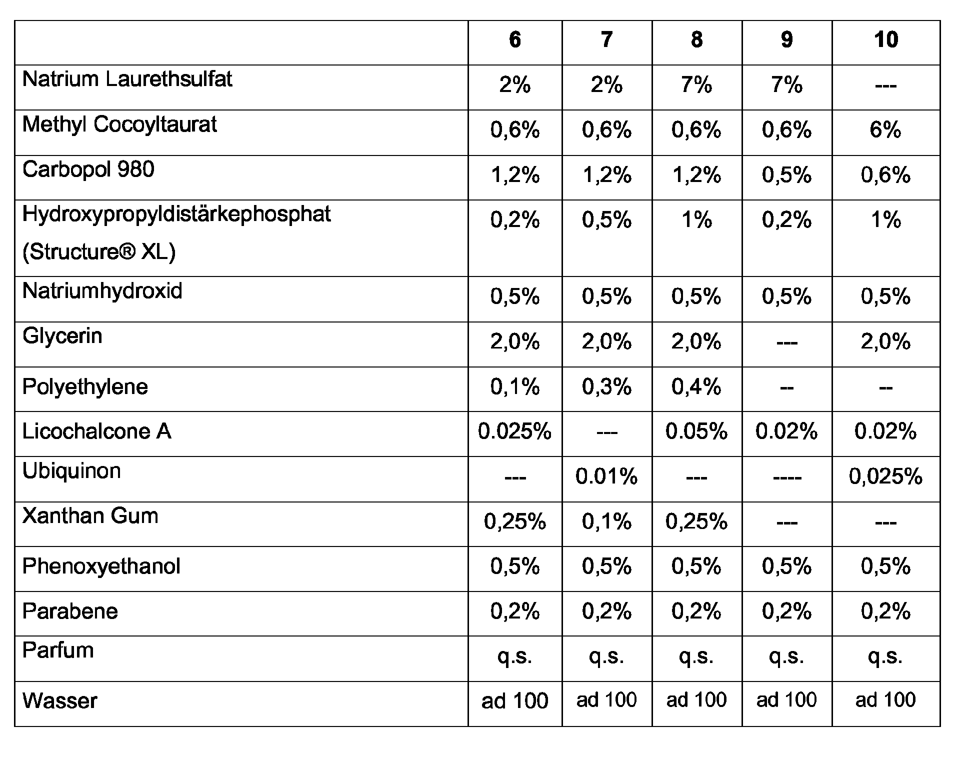

## Patentansprüche

1. Kosmetische Zubereitung enthaltend ein oder mehrere labile Inhaltsstoffe sowie ein oder mehrere vorgelatinisierte, quervernetzte Stärkederivate.

2. Kosmetikum, welches gebildet wird aus
a) einer transparenten oder transluzenten Verpackung und
b) einer kosmetischen Zubereitung enthaltend einen oder mehrere labile Inhaltsstoffe sowie ein oder mehrere vorgelatinisierte, quervernetzte Stärkederivate.

3. Verwendung von vorgelatinisierten, quervernetzten Stärkederivaten zur Stabilisierung von labilen Inhaltsstoffen in Kosmetika.

4. Kosmetikum oder Verwendung nach einem der vorhergehenden Ansprüche, **dadurch gekennzeichnet, dass** als vorgelatinisierte, quervernetzte Stärkederivate hydroxypropylierte Phosphatester der Stärke eingesetzt werden.

5. Kosmetikum oder Verwendung nach einem der vorhergehenden Ansprüche, **dadurch gekennzeichnet, dass** als vorgelatinisierte, quervernetzte Stärkederivate Hydroxypropyldistärkephosphat eingesetzt wird.

6. Kosmetikum oder Verwendung nach einem der vorhergehenden Ansprüche, **dadurch gekennzeichnet, dass** die vorgelatinisierten, quervernetzten Stärkederivate in einer Gesamtkonzentration von 0,05 bis 5 Gewichts-%, bezogen auf das Gesamtgewicht der Zubereitung, in dieser enthalten sind.

7. Kosmetikum oder Verwendung nach einem der vorhergehenden Ansprüche, **dadurch gekennzeichnet, dass** als labile Inhaltsstoffe Licochalkon A und/oder Ubichinon Q-10 eingesetzt werden.

8. Kosmetikum oder Verwendung nach einem der vorhergehenden Ansprüche, **dadurch gekennzeichnet, dass** der die Gesamtmenge an Licochalkon A und Ubichinon Q-10 in der Zubereitung von 0,001 bis 0,3 Gewichts-%, bezogen auf das Gesamtgewicht der Zubereitung, beträgt.

9. Kosmetikum oder Verwendung nach einem der vorhergehenden Ansprüche, **dadurch gekennzeichnet, dass** es sich bei den labilen Inhaltsstoffen um Parfümstoffe handelt.

10. Kosmetikum oder Verwendung nach einem der vorhergehenden Ansprüche, **dadurch gekennzeichnet, dass** der die Gesamtmenge an Parfümstoffen in der Zubereitung von 0 bis 2 Gewichts-%, bezogen auf das Gesamtgewicht der Zubereitung, beträgt.

11. Kosmetikum oder Verwendung nach einem der vorhergehenden Ansprüche, **dadurch gekennzeichnet, dass** es sich bei der kosmetische Zubereitung um ein Reinigungsgel handelt.
